Europäisches Patentamt

(19) European Patent Office

Office européen des brevets

(11) Publication number: **0 110 861**
**B1**

# (12) EUROPEAN PATENT SPECIFICATION

(45) Date of publication of patent specification: **30.07.86**

(51) Int. Cl.⁴: **C 07 C 121/32**

(21) Application number: **83870122.5**

(22) Date of filing: **18.11.83**

(54) **Process for removal of acrolein from acrylonitrile product streams.**

(30) Priority: **22.11.82 US 443356**

(43) Date of publication of application:
**13.06.84 Bulletin 84/24**

(45) Publication of the grant of the patent:
**30.07.86 Bulletin 86/31**

(84) Designated Contracting States:
**DE FR GB IT NL**

(56) References cited:
**GB-A- 821 958**
**GB-A-1 379 090**
**US-A-3 257 445**

(73) Proprietor: **MONSANTO COMPANY**
**Patent Department 800 North Lindbergh Boulevard**
**St. Louis, Missouri 63166 (US)**

(72) Inventor: **Denicola, Thomas Kevin**
**2002 San Sebastian**
**Houston Texas 77058 (US)**

(74) Representative: **McLean, Peter et al**
**Monsanto Europe S.A. Patent Department**
**Avenue de Tervuren 270-272 Letter Box No 1**
**B-1150 Brussels (BE)**

Courier Press, Leamington Spa, England.

## Description

### Background of the invention

This invention relates to the purification of acrylonitrile. More particularly, it relates to removal of acrolein from crude acrylonitrile product streams by localized pH control in the acrylonitrile recovery column.

Acrylonitrile is a well known article of commerce widely used in the manufacture of synthetic resins and fibers. It is also a valuable intermediate in the synthesis of many organic compounds. In most applications, it is essential that the acrylonitrile be in as pure a state as possible because even minute traces of impurities are often a direct cause of either extremely low yields of the desired products or inferior qualities in the end product. This is particularly true when acrylonitrile is used in the preparation of synthetic resins and fibers. In most of the processes for producing this nitrile, minor amounts of carbonyl-containing compounds such as methyl vinyl ketone, acetone, acrolein, methacrolein, crotonaldehyde, acetaldehyde, biacetyl, and other similar carbonyl compounds are simultaneously produced. The presence of even very small quantities of such impurities renders the acrylonitrile unsuitable for many applications. For example, any acrolein present copolymerizes and tends to cross-link with acrylonitrile when the nitrile is polymerized for eventual end-use as a fiber. Thus, it is evident that it is imperative that acrolein be essentially completely removed from the acrylonitrile of commerce if it is to be successfully marketed.

In the preparation of acrylonitrile the crude liquid acrylonitrile product from the absorber is usually passed into a recovery or extractive distillation column where the acrylonitrile is separated from most of the minor amounts of by-products produced, such as acrolein. Prior art methods for removal of acrolein from the crude acrylonitrile stream have involved pH adjustments at various points prior to the recovery column. In U.S. 3,185,636 to Stevens et al, the absorber column pH is maintained at a substantially neutral or slightly alkaline pH, such as a pH of 6.5 to 8.5, whereby the saturated carbonyl compounds in the reactor effluent combine with the excess hydrogen cyanide to form the corresponding cyanhydrin. The patentee, at Column 1, lines 23—32, states that separation of trace amounts of unsaturated carbonyl compounds, such as acrolein, is no problem since they are homo-polymerizable under the normal distillation conditions and require no treating step. However, no indication as to what level is considered trace amounts is given and the pH of the nitrile solution is 5 or less during distillation. Attempts have also been made to separate acrylonitrile and hydrogen cyanide from acrolein and other by-products by extractive distillation using multiple stages of distillation. In U.S. 3,328,266 to Modiano et al the crude acrylonitrile stream from the absorber with a pH generally between 9 and 9.5 is subjected to extractive distillation apparently without any further adjustment of pH. The acrolein content remains high, however, even after the third stage of the distillation is completed and does not produce acrylonitrile of sufficient purity for fiber production where the final acrylonitrile product should preferably contain less than 10 parts per million of acrolein.

The separation of acrylonitrile from acetonitrile and small quantities of acrolein is discussed in U.S. 3,459,639 to Borrel et al. In this patent the distillation recovery column is operated at a pH of at least five and preferably 5—7 at the top of the column and between 9 and 12 at the bottom of the column. Under these conditions the acrolein was not significantly affected and remained above 200 parts per million in the separated organic product phase. And in U.S. 3,462,477 to Caporali et al a pH of between 7.5 and 11 was required to separate the acrolein from the crude acrylonitrile by distillation.

Accordingly, this invention provides a process for the removal of sufficient minor amounts of acrolein from crude acrylonitrile product streams to result in reduction of the acrolein content to less than about 50 and preferably less than 10 parts per million.

### Summary of the invention

The invention provides a process for removal of acrolein from a crude acrylonitrile product stream in a recovery column characterized by maintaining the pH in the zone of maximum acrolein concentration of the recovery column at from 5.25 to 7. The acrolein content in the acrylonitrile product stream leaving the recovery column is thereby reduced to less than about 50 parts per million.

### Description of preferred embodiments

In accordance with this invention it has been found that control of recovery column pH in the range of 5.25 to 7 in the zone or region of maximum acrolein concentration will result in significant reduction in the amount of acrolein present in the crude acrylonitrile stream leaving the recovery column. The zone of maximum acrolein concentration as determined by recovery column tray sample analysis will vary depending upon the nature of the ammoxidation process. In general, the zone will occur in the bottom one-third of the recovery column when the crude acrylonitrile stream from the absorber is fed into the recovery column at about the mid-point as in the usual practice. The zone will normally peak within a small number of trays, for example in a 70-tray distillation recovery column the zone of maximum acrolein concentration may peak between about tray 12 and tray 18.

### Description of the preferred embodiments

A preferred process of the invention is one for reduction of the acrolein concentration in a crude acrylonitrile product stream in a recovery column to less than 20 ppm, characterized by maintaining the pH in the zone of maximum acrolein concentration in the lower third of the recovery column at from 5.25 to 7, withdrawing a bottom portion of the product stream enriched in acrolein or acrolein reaction products from the recovery column and removing a top portion of the product stream from the recovery column, said top portion containing less than 10 ppm acrolein.

In the practice of the invention the pH of the recovery column is maintained and controlled at from 5.25 to 7, preferably from 5.5 to 6.5, by addition of any suitable basic compound to the recovery column in the zone of maximum acrolein concentration. Suitable compounds for maintaining the desired pH include alkali and alkaline earth metal hydroxides such as sodium hydroxide, lithium hydroxide, calcium hydroxide and strontium hydroxide, nitrogen compounds such as ammonium hydroxide, amines, carbonate compounds such as calcium carbonate or any other compound which is compatible with the acrylonitrile process. Sodium hydroxide is preferred.

It has been found that maintaining the pH at from 5.25 to 7, preferably 5.5 to 6.5, in the zone of maximum acrolein concentration is necessary in order to significantly reduce the amount of acrolein in the crude acrylonitrile product stream. The pH of the crude acrylonitrile product stream entering the recovery column will, of course, to a large extent determine the top to bottom pH range along with the concentration of the various stream components. The concentration of acrolein in the recovery column ranges from about 50 parts per million (ppm) in the liquid phase at the bottom of the column to 3 to 6 mole % in the liquid phase at the top of the recovery column. The temperature usually will range from about 110 to 115°C at the bottom to from about 82 to 88°C at the top of the recovery column.

Within the recovery column these conditions of pH, temperature and concentration of stream components which are present will cause a number of competing reactions to occur within the column if an attempt is made to remove acrolein by addition of base to the recovery column feed line. It is known that acrolein can be removed by a base catalyzed reaction with HCN to form the cyanohydrin, betacyanopropionaldehyde, and ultimately other heavy organic compounds which are removed with the recovery column bottoms stream. However, when a base is added to the recovery column feed line for this purpose the pH change which occurs causes the acrylonitrile to also react with the HCN to form undesirable succinonitrile. This problem is avoided by the present invention. By localizing the addition of base to the zone of maximum acrolein concentration in the recovery column the resulting high pH is confined to this zone where the reaction between HCN and acrolein will be at its peak and acrylonitrile concentrations are relatively low. The pH in other areas of the recovery column remains low enough so that the reaction of HCN with acrylonitrile to form succinonitrile is substantially reduced.

The following examples are intended to illustrate the invention more fully but are not intended as limitative.

Example 1

In this example three test runs were conducted in an attempt to reduce the acrolein (ACR) concentration in the crude acrylonitrile (AN) product stream exiting overhead from the recovery column. The column operated with a temperature gradient ranging from approximately 73° at the bottom to 83°C at the top with a pressure of 13.8 kPa (2 psig). The pH of the recovery column was adjusted to 6.5 by injection of 100% sodium hydroxide at three different locations, i.e., into the crude acrylonitrile product stream from the absorber just before the stream enters the recovery column at the midpoint, into the recovery column solvent water immediately prior to the point at which the solvent water enters the top of the recovery column and directly into the bottom half of the recovery column at tray 18 of 40 trays. The pH at tray 18 was 6.5. The feed stream entering the recovery column contained 229 ppm acrolein. The results are shown in Table 1.

TABLE 1

| NaOH Injection point | ppm ACR in AN overhead stream | Recovery column bottom pH | NaOH* usage |
|---|---|---|---|
| Absorber Stream | 88 | 4.95 | .0115 |
| Solvent Water | 78 | 4.6 | .009 |
| Tray 18 | 7 | 6.3 | .013 |

* kg of 100% NaOH per kg of AN in reactor effluent

It is evident from these results that reduction in the ACR content to the desired less than 10 ppm level is possible only when the pH is maintained above 5.0 in the critical lower portion of the recovery column where the ACR concentration is high. Monitoring of the recovery column bottom pH shows that the pH drops below 5.0 if either the absorber stream or the solvent water are used as the pH control point.

Example 2

A series of runs were conducted using a standard 70 tray acrylonitrile (AN) recovery column. A sufficient amount of the indicated hydroxides was injected into the column at tray 12 where the acrolein (ACR) concentration, as determined by analysis of tray samples, was the highest, i.e., 1042 ppm, to maintain the column pH at the indicated levels. The acrolein and succinonitrile (SN) concentrations in parts per million (ppm) in the crude AN stream passing through the column were measured at the bottom and top of the column.

For comparative purposes in Run 5 the hydroxide was added to the recovery column feed line from the absorber. The results are reported in Table 2 below.

TABLE 2

| Run No. | Hydroxide | pH | ACR ppm | | SN ppm | | Hydroxide usage |
|---|---|---|---|---|---|---|---|
| | | | Bottom | Top | Bottom | Top | |
| 1 | None | 5.0 | 136 | 150 | 4687 | 1681 | — |
| 2 | 5% NaOH | 5.5 | 53 | 32 | 5843 | 853 | .0157 |
| 3 | 5% NaOH | 5.0 | 160 | 179 | 4222 | 899 | .0116 |
| 4 | 5% NH$_4$OH | 5.5 | 61 | 5 | 7412 | 544 | — |
| 5 | 5% NaOH | 5.5 | 78 | 19 | 16461 | 305 | — |

These runs show that both the pH and the addition point in the column are critical. Runs 1 and 3 demonstrate that at a low pH of 5.0 the ACR remains high with or without addition of hydroxide. Run No. 5 indicates that pH control by addition of hydroxide into the recovery column feed line instead of at the point of maximum ACR concentration is undesirable since although ACR is low SN increases to more than three times normal. This is apparently due to the fact that the high concentrations of HCN and AN in the upper sections of the recovery column caused increased SN formation when hydroxide is added to the feed line near the midpoint of the column. Thus, the localized area of high pH, about 5.25 or higher, where it is needed to react the ACR at its maximum concentration is created by addition of hydroxide at the point of maximum ACR concentration. The pH in other areas of the recovery column can be kept low enough, around 5.0, so that SN formation is not a problem. A pH above about 7 in the zone of maximum ACR concentration is also undesirable since this will also tend to increase undesirable reactions such as SN formation.

The examples also indicate that the amount and the concentration of alkali or alkaline earth hydroxide needed to maintain the desired pH range will vary widely depending upon the conditions in the recovery column. Based on the amount of acrylonitrile produced the hydroxide may be used in amounts ranging from about .001 to .020 kg per kg of acrylonitrile in the reactor effluent. The concentration will vary from less than about 5% to 100% with the choice of concentration merely a matter of convenience or availability.

The foregoing detailed description has been given primarily for clearness of understanding only and unnecessary limitations are not to be construed therefrom. The invention is not to be limited to the exact details shown and described since obvious modifications will occur to those skilled in the art.

**Claims**

1. A process for removal of acrolein from a crude acrylonitrile product stream in a recovery column characterized by maintaining the pH in the zone of maximum acrolein concentration of the recovery column at from 5.25 to 7.

2. The process of Claim 1 wherein the pH is from 5.5 to 6.5.

3. The process of Claim 1 wherein the zone of maximum acrolein concentration is in the lower third of the recovery column.

4. The process of Claim 1 wherein the pH is maintained by injection of an alkali or alkaline earth metal hydroxide into said zone.

5. The process of Claim 4 wherein the alkali metal hydroxide is sodium hydroxide.

6. The process of Claim 1 wherein the pH is maintained by injection of ammonium hydroxide.

7. A process for reduction of the acrolein concentration in a crude acrylonitrile product stream in a recovery column to less than 20 ppm characterized by maintaining the pH in the zone of maximum acrolein concentration in the lower third of the recovery column at from 5.25 to 7, withdrawing a bottom portion of the product stream enriched in acrolein or acrolein reaction products from the recovery column and removing a top portion of the product stream from the recovery column, said top portion containing less than 10 ppm acrolein.

8. The process of Claim 7 wherein the pH is from 5.5 to 6.5.

9. The process of Claim 7 wherein the pH is maintained by injection of an alkali or alkaline earth metal hydroxide into said zone.

10. The process of Claim 9 wherein the alkali metal hydroxide is sodium hydroxide.

**Patentansprüche**

1. Verfahren zur Abtrennung von Acrolein aus einem rohen Acrylnitrilproduktstrom in einer Rückgewinnungskolonne, dadurch gekennzeichnet, daß der pH-Wert in der Zone der maximalen Acroleinkonzentration der Rückgewinnungskolonne bei 5,25 bis 7 gehalten wird.

2. Verfahren nach Anspruch 1, dadurch gekennzeichnet, daß der pH-Wert bei 5,5 bis 6,5 gehalten wird.

3. Verfahren nach Anspruch 1, dadurch gekennzeichnet, daß die Zone maximaler Acroleinkonzentration im unteren Drittel der Rückgewinnungskolonne liegt.

4. Verfahren nach Anspruch 1, dadurch gekennzeichnet, daß der pH-Wert durch Einführen eines Alkalimetall- oder Erdalkalimetallhydroxids in die Zone aufrechterhalten wird.

5. Verfahren nach Anspruch 4, dadurch gekennzeichnet, daß als Alkalimetallhydroxid Natriumhydroxid verwendet wird.

6. Verfahren nach Anspruch 1, dadurch gekennzeichnet, daß der pH-Wert durch Einführen von Ammoniumhydroxid aufrechterhalten wird.

7. Verfahren zur Verminderung der Acroleinkonzentration in einem rohen Acrylnitrilproduktstrom in einer Rückgewinnungskolonne auf einen Wert von weniger als 20 ppm, dadurch gekennzeichnet, daß der pH-Wert in der Zone maximaler Acroleinkonzentration im unteren Drittel der Rückgewinnungskolonne bei 5,25 bis 7 gehalten wird, ein Sumpfanteil des an Acrolein oder Acroleinreaktionsprodukten angereicherten Produktstroms aus der Rückgewinnungskolonne abgezogen und ein Kopfanteil des Produktstroms aus der Rückgewinnungskolonne abgetrennt werden, wobei der Kopfanteil weniger als 10 ppm Acrolein enthält.

8. Verfahren nach Anspruch 7, dadurch gekennzeichnet, daß der pH-Wert bei 5,5 bis 6,5 gehalten wird.

9. Verfahren nach Anspruch 7, dadurch gekennzeichnet, daß der pH-Wert durch Einführen eines Alkalimetall- oder Erdalkalimetallhydroxids in die Zone aufrechterhalten wird.

10. Verfahren nach Anspruch 9, dadurch gekennzeichnet, daß als Alkalimetallhydroxid Natriumhydroxid eingesetzt wird.

**Revendications**

1. Procédé d'élimination de l'acroléine d'un courant de produit d'acrylonitrile brut dans une colonne de récupération, caractérisé en ce qu'on maintient le pH dans la zone de concentration maximum en acroléine de la colonne de récupération à une valeur de 5,25 à 7.

2. Procédé selon la revendication 1, dans lequel le pH est de 5,5 à 6,5.

3. Procédé selon la revendication 1, dans lequel la zone de concentration maximum en acroléine est dans le tiers inférieur de la colonne de récupération.

4. Procédé selon la revendication 1, dans lequel le pH est maintenu par injection d'un hydroxyde de métal alcalin ou alcalino-terreux dans cette zone.

5. Procédé selon la revendication 4, dans lequel l'hydroxyde de métal alcalin est l'hydroxyde de sodium.

6. Procédé selon la revendication 1, dans lequel le pH est maintenu par injection d'hydroxyde d'ammonium.

7. Procédé pour la réduction de la concentration en acroléine dans un courant de produit d'acrylonitrile brut dans une colonne de récupération à moins de 20 ppm, caractérisé en ce qu'on maintient le pH dans la zone de concentration maximum en acroléine dans le tiers inférieur de la colonne de récupération à une valeur de 5,25 à 7, en ce qu'on prélève une partie inférieure du courant de produit enrichi en acroléine ou en produits de réaction de l'acroléine de la colonne de récupération, et en ce qu'on élimine une partie supérieure du courant de produits de la colonne de récupération, cette partie supérieure contenant moins de 10 ppm d'acroléine.

8. Procédé selon la revendication 7, dans lequel le pH est de 5,5 à 6,5.

9. Procédé selon la revendication 7, dans lequel le pH est maintenu par injection d'un hydroxyde de métal alcalin ou alcalino-terreux dans cette zone.

10. Procédé selon la revendication 9, dans lequel l'hydroxyde de métal alcalin est l'hydroxyde de sodium.